# EUROPÄISCHE PATENTANMELDUNG

(11) **EP 2 347 728 A1**
(43) Veröffentlichungstag der Anmeldung: **27.07.2011**
(21) Anmeldenummer: 11000430.6
(22) Anmeldetag: 20.01.2011
(51) Int. Cl.: A61B 19/02

(54) **Deckelbehälter**

(30) Priorität: 21.01.2010 DE 202010001236 U
(71) Anmelder: Mauser-Werke GmbH, 50321 Brühl (DE)
(72) Erfinder: Verduijn, Gijsbert Johannes, 4844 SC Terheijden (NL)

(57) **Zusammenfassung**

Die vorliegende Erfindung betrifft einen besonderen Behälter mit Deckel, die beide aus thermoplastischem Kunststoff hergestellt sind.

Dieser besondere Deckelbehälter ist speziell für eine Verwendung als Abfallbehälter in Krankenhäusern, Veterinär-Kliniken, Arzt- und Zahnarzt-Praxen und medizinischen Forschungs-und Entwicklungslaboratorien insbesondere für infektiöse Abfälle vorgesehen und entsprechend dafür konzipiert. Für eine Sterilisationsbehandlung mit Heißdampf ist der Behälterdeckel mit einem weiteren, darin eingesetzten Schrumpfdeckel ausgestattet.

## Beschreibung

Die vorliegende Erfindung betrifft einen besonderen Deckelbehälter mit Behälterkörper und Behälterdeckel aus thermoplastischem Kunststoff, der für eine Verwendung als Abfallbehälter in Krankenhäusern, Veterinär-Kliniken, Arzt- und Zahnarzt-Praxen und medizinischen Forschungs-und Entwicklungslaboratorien insbesondere für infektiöse Abfälle vorgesehen ist, wobei für eine Sterilisationsbehandlung im Behälterdeckel ein weiterer kleinerer Verschlussdeckel als Schrumpfdeckel vorgesehen ist.

Ein derartiger Behälter mit Schrumpfdeckel ist bereits aus der EP 0 965 353 A1 vorbekannt. Nachteil des bekannten Behälters (EP 0 965 353 A1) sind hohe Energieverluste durch langen Zeitaufwand und erhöhter Energieaufwand für eine Sterilisation bei Temperaturen zwischen 130 und 150 ° C.

Die Abfallbehälter, um die es hierbei geht, weisen in aller Regel eine Größe von etwa 50 bis 60 Liter auf. Ein derartiger Abfallbehälter ist aus der WO 94/13562 (VAT 4) bekannt und dient beispielsweise für ein sicheres und hygienisches Verpacken und Entsorgen von infektiösen Sonderabfällen aus dem Gesundheitswesen (z. B. gebrauchtes Verbandsmaterial).

Die Behandlung, d. h. der Umgang und die Entsorgung von Abfällen aus dem Gesundheitsdienst ist mit Inkrafttreten des Kreislaufwirtschafts- und Abfallgesetz (KrW/AbfG) sowie der Einführung des Europäischen Abfallverzeichnisses (AVV) europarechtskonform geregelt und muss unter Beachtung des Abfall-, Infektions-, Arbeitsschutz-, Chemikalien- und Gefahrgutrechts erfolgen. Krankenhäuser gehören zu den größten kommunalen Müllproduzenten. Ein nicht unerheblicher Teil davon sind invektiöse Abfälle, die nur in besonderen Behältern gesammelt, transportiert und in Sonderabfall-Verbrennungsanlagen verbrannt werden dürfen (teuer).

### Aufgabe:

Die Aufgabe der vorliegenden Erfindung besteht in einer Ermöglichung eines normalen Transportes und normaler Handhabung von kritischen invektiösen Krankenhausabfällen durch Verminderung des Gefahrenrisikos beim Umgang (Einsammeln, Zwischenlagerung, Transport, Entsorgung z. B. Verbrennung, allgemeines Handling) mit den zuvor genannten Sonderabfällen. Ganz wesentlich sind dabei die Aspekte für einen verbesserten Umweltschutz, für eine spürbare Energieeinsparung und insbesondere eine Reduzierung des Risikos für diejenigen Personen, die mit infektiösen Abfallstoffen zu tun haben.

Diese Aufgabe wird erfindungsgemäß mit den Merkmalen des Anspruches 1 gelöst.

Die Unteransprüche zeigen wichtige Merkmale zur weiteren Ausgestaltung dieser Erfindung auf. Die Vorteile des erfindungsgemäßen Deckelbehälters bestehen in :
Beginn der Dampfsterilisierung bei niedrigen Temperaturen (dadurch Verminderung des Energieaufwandes),
   - optimale Sterilisationsbedingungen durch zentrale Anordnung der Dampf-Zutrittsöffnung,
   - gleichmäßige und schnellere Sterilisierung,
Mindestöffnungsweite des Dampfeintrittes,
   - gleiche und kurze Wege des Sterilisationsdampfes,
   - Ermöglichung einer Sterilisation eines befüllten Großbehälters mit einem Fassungsvermögen von 60 Litern bei vergleichsweise niedrigen Temperaturen von ca. 130° C,
   - Wieder-Verschließbarkeit des Behälters (im sterilen Zustand) direkt nach dem Sterilisationsvorgang.

Keine Infektionsgefahr für Personen die mit den befüllten Behältern hantieren müssen. Der gefüllte Behälter mit hochinvektiösem Material muss nicht vom Bedienungspersonal - wie bisher bei üblicherweise verwendeten Metallbehältern erforderlich war - vor dem Einsetzen in den Autoklaven geöffnet werden, um einen Dampfzutritt zu ermöglichen.

### Zweck der Sterilisation ist folgender :

Abfallbehälter mit infektiösem Inhalt werden üblicherweise als Gefahrgutbehälter eingestuft und sind dementsprechend als solche unter höchsten Sicherheitsbedingungen zu transportieren, zu lagern und zu entsorgen. Eine Entsorgung erfolgt durch vollständige Verbrennung des geschossenen Behälters samt Inhalt. Mit der besonderen Desinfektionsbehandlung des erfindungsgemäßen Deckelbehälters in den Krankenhäusern selbst mit eigenem, ausreichend großen Autoklaven wird ermöglicht, dass die Behälter mit dampfbehandeltem Inhalt nicht mehr als kostenintensiver Sondermüll zu behandeln sind. Nachdem ein gefüllter Abfallbehälter mit einem derartig kritischen Inhalt vollständig sterilisiert wurde, und nach Entnahme aus dem Autoklaven sofort mit einem neuen zentralen Einsatzdeckel und neuer Dichtung gasdicht verschlossen wurde, kann er als normaler (ungefährlicher) Abfall transportiert und zwischengelagert werden. Infektiöser Abfall, der desinfiziert bzw. sterilisiert wurde, entspricht nicht-gefährlichem Abfall und kann wie dieser als normaler medizinischer Abfall entsorgt und z. B. in Müllverbrennungsanlagen verbrannt werden. Nach dem erneuten Verschließen des sterilisierten Behälters mit einem neuen zentralen Verschlussdeckel entspricht der Behälter einer UN-zertifizierten Verpackung und kann nach den Gefahrgutvorschriften (ADR-Regularien) transportiert werden. Abfallbehälter mit infektiösem Inhalt, die nicht sterilisiert/desinfiziert sind, dürfen nicht im öffentlichen Straßenverkehr transportiert werden.

Der eingangs genannte bekannte Behälter, der für derartige Zwecke einsetzbar wäre, weist nur sehr kleine Sterilisationsöffnung mit Abschmelzverschluss auf, der erst bei vergleichsweise hohen Temperaturen von ca. 130 °C und mehr aufschmilzt und dann auch nur einen begrenzten Dampfzugang erlaubt. Wegen der vergleichsweise kleinen Öffnung würde eine derartige Sterilisationsbehandlung mit Heißdampf eine vergleichsweise sehr lange Behandlungszeit brauchen, bis der gesamte Inhalt auch in den entferntesten Behälterecken sterilisiert ist, was somit einen hohen Energiebedarf benötigt. Der bekannte Behälter hat in der Praxis keine Anwendung gefunden.

Demgegenüber wird mit der vorliegenden Erfindung der Energiebedarf für die erforderliche Sterilisationsbehandlung in einem Sterilisationsautoklaven erheblich gesenkt. Zum einen erfolgt der Sterilisationsvorgang schon nach erheblich kürzeren Zeiten, da eine Aufwärmung des Behälter-Materials und insbesondere des Schmelzdeckels auf 100° C schnell erfolgt, so dass ein Dampfzutritt in das Behälterinnere bereits ab Temperaturen von ca. 100°C durch die vergleichsweise große Zugangsöffnung (= Sterilisationsöffnung) ermöglicht wird. Dadurch kann dann auch die Aufheizung des Behälterinhaltes auf eine Sterilisationstemperatur von 130° C frühzeitiger und schneller erfolgen. Durch die zentralmittige Anordnung des Schmelzdeckels im großen Behälterdeckel sind in optimaler Weise vergleichsweise kurze bzw. gleichlange Wege für den eindringenden Heißdampf für eine gleichmäßige Sterilisation des gesamtem Behälterinhaltes gewährleistet. Sobald eine vollständige Durchhitzung des Behälterinhaltes durch den Heißdampf auf eine Temperatur von ca. 130° C erfolgt ist, ist die Sterilisation nach wesentlich kürzerer Zeit als bisher erfolgreich beendet; der sterilisierte Behälter wird aus dem Autoklaven entnommen und sofort mit neuem Einsatzdeckel und neuer Dichtung gas- und flüssigkeitsdicht verschlossen, so dass kein Sauerstoff und keine neuen Keime eindringen können. Die manuelle Einschraubung des neuen Verschlussdeckels in die Sterilisationsöffnung stellt für das Bedienungspersonal kein Risiko dar, da zu diesem Zeitpunkt der Behälterinhalt bereits sterilisiert ist.

Die vorliegende Erfindung wird nachfolgend anhand von schematisch in den Zeichnungsfiguren dargestellten Ausführungsbeispielen näher erläutert und beschrieben. Es zeigen :
- Figur 1: einen erfindungsgemäßen Deckelbehälter mit aufgesetztem Behälterdeckel,

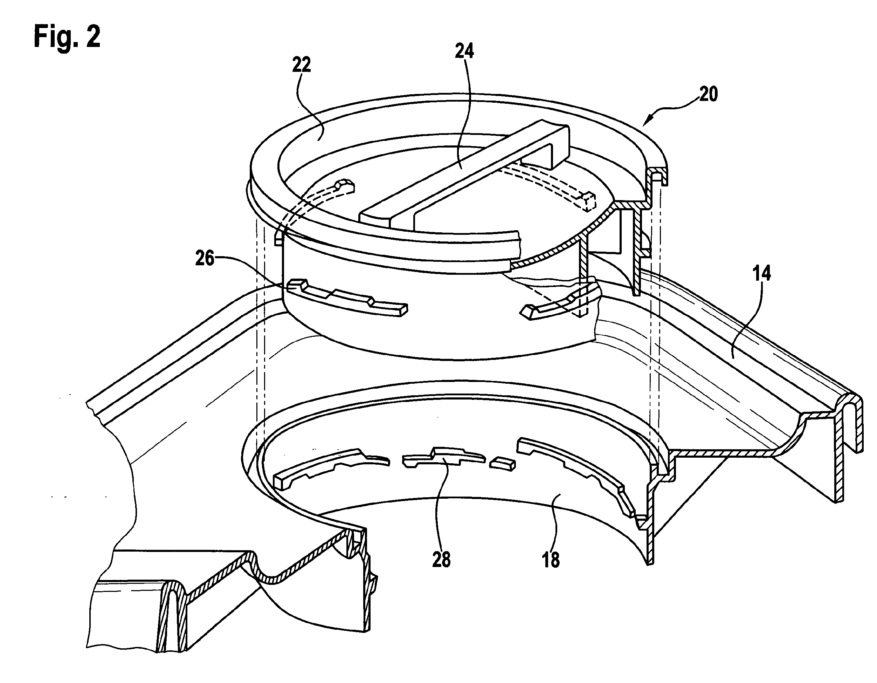

- Figur 2: in Teilschnittdarstellung den Behälterdeckel mit darin angeordnetem runden Verschlussdeckel (= Schrumpfdeckel)

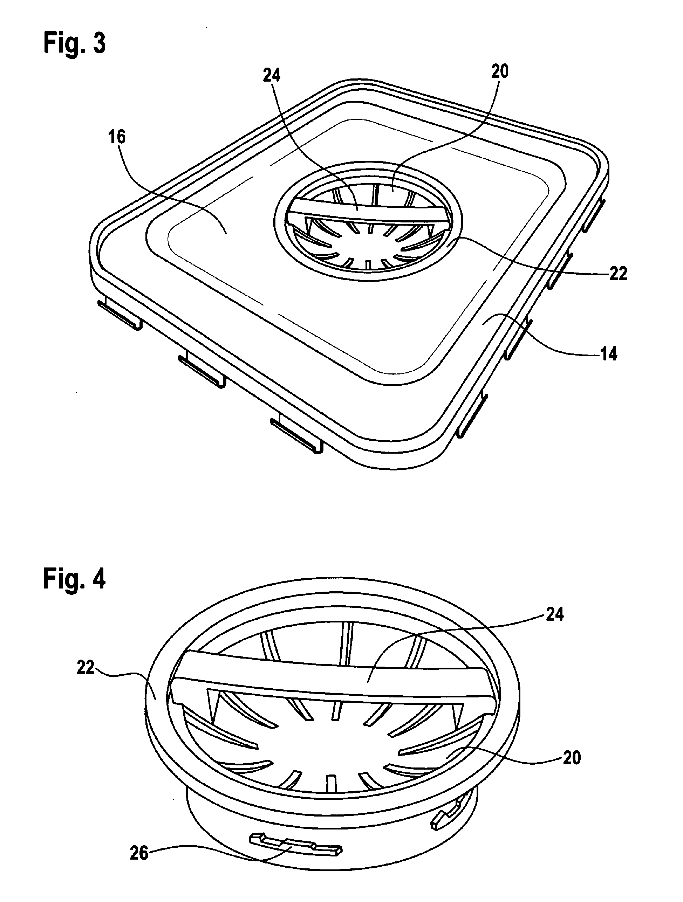

- Figur 3: einen Behälterdeckel gemäß der Erfindung als Schnappdeckel mit einem zentralmittig eingesetzten runden Verschlussdeckel als Schrumpfdeckel,
- Figur 4: den herausgenommenen Schrumpfdeckel,

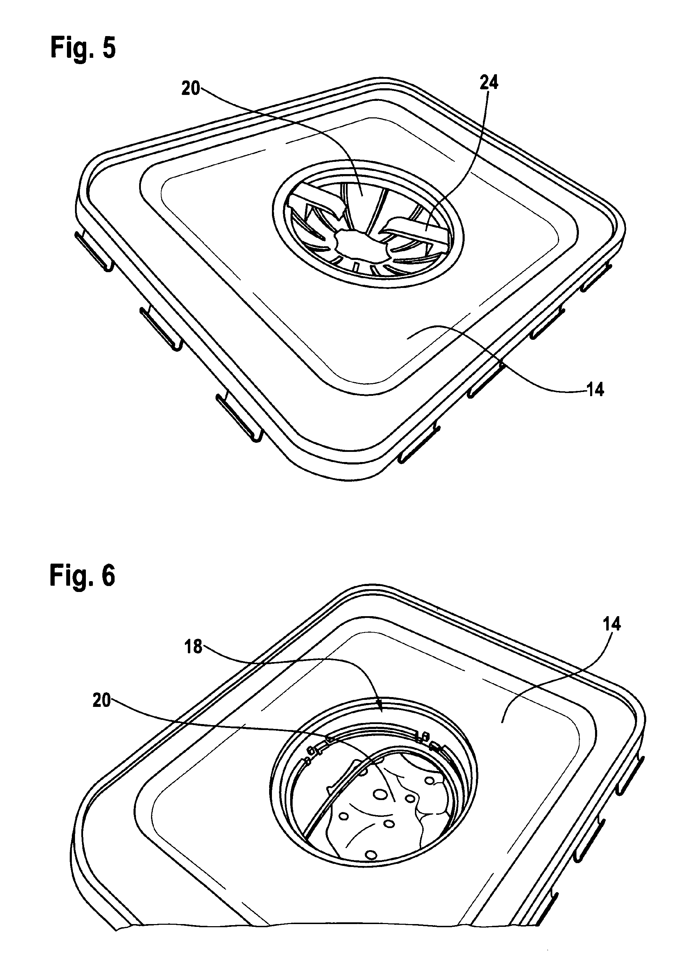

- Figur 5: den Behälterdeckel gemäß Fig. 3 nach kurzem Einsatz in einem Sterilisationsautoklaven mit beginnendem Aufschmelzen des Schrumpfdeckels
- Figur 6: den Behälterdeckel gemäß Fig. 3 nach erfolgtem Einsatz im Sterilisationsautoklaven mit vollständig aufgeschmolzenem hineingefallenem Schrumpfdeckel

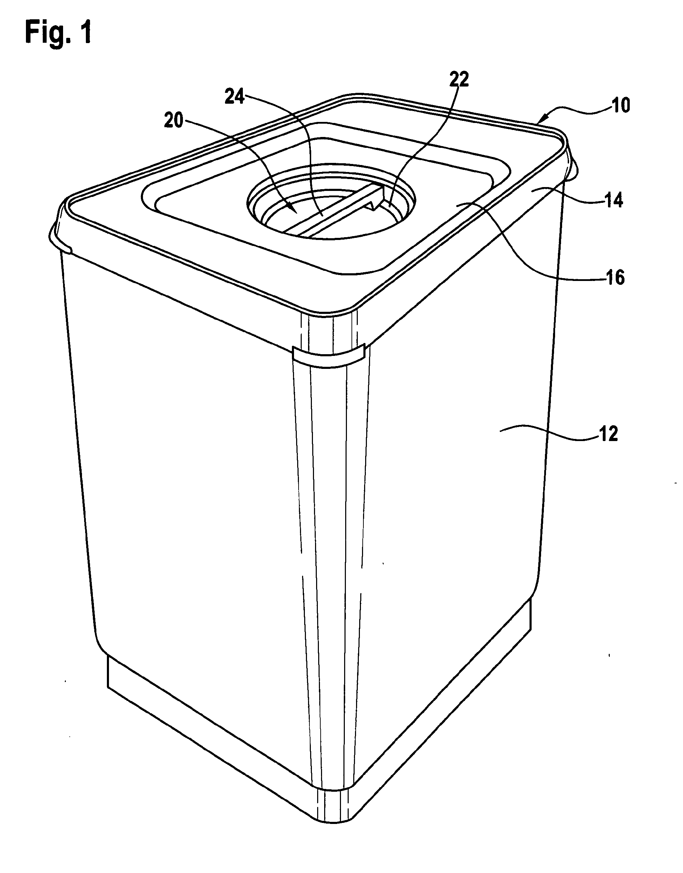

In Figur 1 ist mit der Bezugsziffer 10 ein erfindungsgemäßer Deckelbehälter in Rechteckformat mit auf die Behälteröffnung des Behälterkörpers 12 aufgesetztem Behälterdeckel 14 bezeichnet. Bei einem rechteckförmigen Deckelbehälter in der Größe von 30, 40, 50 bis 60 Litern Fassungsvermögen (lediglich die Behälterhöhe sind hier unterschiedlich von min. 320 mm bis max. ca. 650 mm) betragen die Abmessungen der Behälteröffnung bzw. des gleichfalls rechteckförmigen Behälterdeckels ca. 320 x 380 mm.

Der Behälterdeckel 14 ist als Schnappdeckel ausgebildet und kann nach festem Aufpressen auf die Behälteröffnung nach Einrasten einer Vielzahl von hakenförmigen Verschlusselementen am Deckelrand in entsprechenden Schlitzen an der Außenseite der Behälteröffnung am oberen Behälterkörper 12 nicht mehr vom Behälterkörper 12 abgenommen werden. Es könnte aber auch ein normaler Deckel mit Spannringverschluss aufgesetzt sein.

Im mittleren Bereich des Behälterdeckels 14 ist eine rechteckförmige Deckelfläche 16 etwas eingesenkt bzw. vertieft ausgebildet und weist in diesem Bereich, d. h. zentralmittig in der Deckelfläche des Behälterdeckels 14 eine weitere kreisrunde Öffnung 18 mit entsprechendem Verschlussdeckel 20 auf.

Dieser runde Verschlussdeckel 20 besteht aus einem besonderen Kunststoffmaterial, das unter Hitzeeinwirkung einschrumpft und sich zusammenzieht (= → Schrumpfdeckel).

Das für den Schrumpfdeckel vorgesehene Material kann z. B. aus einem besonderen Ethylen-Vinyl Acetat Co-polymer bestehen, das eine Schmelztemperatur von ca. 98° C aufweist und z. B. im Spritzgussverfahren verarbeitet werden kann. Ein solches Material wird z. B. von der Fa. Du Pont unter der Handelsbezeichnung Elvax 760A vertrieben.

Der Behälterkörper, Behälterdeckel und der Schrumpfdeckel werden im Spritzgussverfahren aus normalem handelsüblichem HDPE (High Density - Poly Ethylen) hergestellt; der Behälterkörper kann auch im Blasformverfahren hergestellt werden.

Der in vorteilhafter Weise rund ausgebildete Verschlussdeckel 20 weist - wie auch die kreisrunde Öffnung 18 - einen Durchmesser von ca. 90 mm bis 150 mm, vorzugsweise ca. 120 mm auf und ist über deckelseitige Verriegelungselemente 26 und in der Öffnung 18 vorgesehene Verriegelungselemente 28 fest, d. h. gas- und flüssigkeitsdicht mittels eines eingesetzten Dichtungsringes, in die Öffnung 18 eingesetzt. Die Behälteröffnung 18 könnte in entsprechender Größe natürlich auch rechteckig oder quadratisch gestaltet sein; wichtig ist der große Öffnungsquerschnitt der Begasungsöffnung für einen optimalen Dampfzutritt von wenigstens 60 qcm (6.000 qmm). So kann auch die Befüllung des Behälters selbst durch diese vergleichsweise große zentralmittige Öffnung 19 erfolgen.

Durch die Größe, die konstruktive dünnwandige Beschaffenheit und das frühzeitige Schmelzverhalten des Schrumpfdeckel wird eine schnelle Öffnung der Begasungsöffnung sichergestellt, so dass es bei der Aufwärmung des Kunststoffbehälters zu keinen nachteiligen Deformationen des Deckelbehälters selbst kommt, was der Fall wäre, wenn das eingeschlossene Luftvolumen nicht frühzeitig entweichen könnte.

Für eine weitere Beschleunigung der Sterilisationsbehandlung ist vorgesehen, dass der Verschlussdeckel 20 (Schrumpfdeckel) für einen Deckelbehälter in der Volumengröße von ca. 60 Litern einen Durchmesser von ca. 100 mm bis 150 mm, vorzugsweise ca. 140 mm, aufweist. Ein großer Schrumpfdeckel beginnt früher zu schrumpfen als ein kleiner und öffnet demnach auch früher die Begasungsöffnung.

Während des Sterilisationsvorganges wird oftmals ein "Pumpen" des Heißdampfes eingestellt, so dass ein Durchspülen des Behälterinhaltes erfolgt, wodurch die erforderliche Zeit zur vollständigen Sterilisation/Desinfektion verkürzt wird. Dabei wirkt sich die Größe der Begasungsöffnung ebenfalls förderlich aus.

Nach Einstellen eines mit Krankenhausabfällen gefüllten Deckelbehälters in einen Sterilisationsautoklaven und Einwirken der Dampftemperatur von kurz über 100° C beginnt der Verschlussdeckel 20 alsbald aufzuschmelzen und schrumpft im Durchmesser derart ein (zieht sich zusammen), dass er nach kurzer Zeit in den Behälter hinein auf das Abfallgut fällt (und dort verbleibt und mit entsorgt wird). Nun ist die weitere kreisrunde Öffnung 18 vollkommen frei für einen ungehinderten Durchtritt des Sterilisationsdampfes für eine zügige Sterilisation des Füllgutes. Nach vollständig durchgeführter Sterilisation wird der sterilisierte Deckelbehälter aus dem Sterilisationsautoklaven herausgenommen und die kreisrunde Öffnung 18 wird unverzüglich mit einem neuen Verschlussdeckel 20 und mit einer neuen Dichtung gas- und flüssigkeitsdicht verschlossen. Dadurch wird ein erneutes Eindringen von Keimen, Bakterien, Vieren und Sauerstoff wirksam verhindert.

Der neue Verschlussdeckel 20 besteht aus normalem Kunststoff (z. B. preiswertes HDPE) und ist nicht als Schrumpfdeckel ausgebildet. Der sterilisierte Deckelbehälter mitsamt sterilisiertem Inhalt kann nun als nicht-gefährlicher, normaler medizinischer Abfall gehandhabt und entsorgt werden.

Mit einem Einsatz bzw. bei einer Verwendung des erfindungsgemäßen Deckelbehälters werden auf einfache und vorteilhafte Weise bei der problematischen Behandlung von Sonderabfällen wirkungsvolle Maßnahmen für einen verbesserten Umweltschutz und für eine spürbare Einsparung von Energie bei der Aufheizung des Deckelbehälters und Sterilisation des Inhaltes aufgezeigt, die eine erhebliche Verminderung von Zeit- und Kostenaufwand bewirken und insbesondere auch diejenigen Personen schützt, die mit gefährlichen invektiösen medizinischen Abfällen in Berührung kommen.

### Bezugsziffernliste

- 10: Deckel-Behälter
- 12: Behälterkörper
- 14: Behälterdeckel
- 16: zentrale eingesenkte Deckelfläche
- 18: kreisrunde Öffnung
- 20: Verschlussdeckel
- 22: oberer Flanschrand
- 24: mittiger Griff
- 26: Verriegelungselemente (20)
- 28: Verriegelungselemente (18,14)

## Patentansprüche

1. Deckelbehälter (10) mit Behälterkörper (12) und Behälterdeckel (14) aus thermoplastischem Kunststoff, der für eine Verwendung als Abfallbehälter in Krankenhäusern, Veterinärkliniken, Arzt- und Zahnarzt-Praxen und medizinischen Forschungs- und Entwicklungslaboratorien insbesondere für infektiöse Abfälle vorgesehen ist, wobei für eine Sterilisationsbehandlung im Behälterdeckel (14) ein weiterer kleinerer Verschlussdeckel (20) als Schrumpfdeckel vorgesehen ist,
**dadurch gekennzeichnet, dass**
eine für den Einsatz des weiteren Verschlussdeckels (20) zentralmittig im Behälterdeckel (14) vorgesehene kreisrunde Öffnung (18) einen Durchmesser von 90 mm bis 150 mm, vorzugsweise ca. 120 mm aufweist.

2. Deckelbehälter nach Anspruch 1,
**dadurch gekennzeichnet, dass**
der als Schrumpfdeckel ausgebildete Verschlussdeckel (20) aus einem besonderen Kunststoff-Werkstoff mit einer niedrigen Erweichungs-Schmelztemperatur von ca. 95° C -110° C, vorzugsweise ca. 100° C, besteht und im Spritzgussverfahren hergestellt ist.

3. Verwendung eines Deckelbehälters nach Anspruch 1 oder 2 für eine Sterilisierung von insbesondere infektiösem Behälterinhalt,
**dadurch gekennzeichnet, dass**
ein mit infektiösen Abfällen gefüllter Deckelbehälters aus thermoplastischem Kunststoff vollständig verschlossen in einen Sterilisations-Autoklaven eingestellt und einer Sterilisierungsbehandlung bei Einwirkung einer Dampftemperatur von ca. 130° C unterworfen wird, wobei der Verschlussdeckel (20) bereits bei einer Temperatur von ca. 100° C aufschmilzt und derart im Durchmesser einschrumpft, so dass er nach kurzer Zeit in den Behälter hinein auf das Abfallgut fällt, so dass die kreisrunde Öffnung mit einem Öffnungsquerschnitt von wenigstens 60 qcm vollkommen frei für einen ungehinderten Durchtritt des Sterilisationsdampfes ist und eine zügige Sterilisation des Füllgutes erfolgt, wobei nach vollständig durchgeführter Sterilisation der sterilisierte Deckelbehälter aus dem Sterilisationsautoklaven herausgenommen und die kreisrunde Öffnung unverzüglich mit einem neuen Verschlussdeckel (kein Schmelzdeckel) gas-und flüssigkeitsdicht verschlossen wird.
